# EUROPEAN PATENT SPECIFICATION

(11) **EP 1 890 670 B1**
(45) Date of publication and mention of the grant of the patent: **29.06.2011**
(21) Application number: 05779006.5
(22) Date of filing: 03.08.2005
(51) Int. Cl.: A61K 8/00, A61K 8/99, A61Q 5/06

(54) **POLYSACCHARIDE GRAFT COPOLYMERS AND THEIR USE IN HAIR CARE APPLICATION**
POLYSACCHARID-PFROPFCOPOLYMERE UND IHRE VERWENDUNG BEI DER HAARPFLEGE
COPOLYMERES GREFFES DE POLYSACCHARIDE ET LEUR UTILISATION DANS DES APPLICATIONS DE SOINS DE LA CHEVÈLURE

(30) Priority: 03.08.2004 US 598548 P
(43) Date of publication of application: 27.02.2008
(73) Proprietor: Rhodia, Inc., Cranbury, NJ 08512 (US)
(72) Inventor: GUNN, Euen, Trenton, NJ 08611 (US); LIU, Leo Zhaoqing, Lawrenceville, NJ 08648 (US); PRIOU, Christian, 69260 Charbonnieres Les Bains (FR); GABBIANELLI, Alvino, Holland, PA 18966-2831 (US); WARBURTON, Stewart Alexander, West Windsor, NJ 08550-3519 (US)
(74) Representative: Russell, Lindsey
(86) International application number: PCT/US2005/027597
(87) International publication number: WO 2006/017589

(56) References cited:
- US-A- 4 810 567
- US-A- 5 801 116
- US-A- 5 807 543
- US-A1- 2004 072 947
- US-B1- 6 649 155

## Description

### Field of the Invention

This invention relates to graft copolymers, more particularly polysaccharide graft copolymers, and their use in personal care compositions.

### Background of the Invention

Many hair care products contain conditioning, volumizing and styling agents, which are typically high molecular weight polymers with an inherent attraction to hair surfaces are known (US 2004/0072947; US 6,649,155). These polymers may be either derived from synthetic or natural sources. These polymers may also function as adjuvants in the delivery of supplemental actives such as silicones.

Often polymers that provide hair care benefits also provide skin care benefits such as conditioning and/or feel.

There is a continuing interest in the art in developing hair conditioning agents that provide high conditioning, volumizing, or styling performance.

### Summary of the Invention

In a first aspect, the present invention is directed to a graft copolymer comprising one or more blocks of a polymer capable of bonding to human hair and blocks of one or more polymers that are not capable of bonding with human hair grafted to a polysaccharide polymer backbone, wherein the polymer capable of bonding to human hair is a cationic polymer, or a substantially neutral polymer capable of forming hydrogen bonds with hair.

In a second aspect, the present invention is directed to a method for making a graft copolymer comprising:
mixing the polysaccharide polymer, the polymer capable of bonding to human hair, and the polymer not capable of bonding with human hair; and
irradiating the polymer mixture with electron beam irradiation to form the graft copolymer.

In a third aspect, the present invention is directed to a hair care composition, comprising the above-described graft copolymer.,.

### Detailed Description of Invention and Preferred Embodiments

As used herein in reference to a graft copolymer, the term "backbone" means the main polymeric chain of a molecule of the graft copolymer.

As used herein, the term "block" in reference to a graft copolymer means a portion of the graft copolymer molecule that has at least one constitutional or configurational feature that differs from that of the backbone of the graft copolymer.

As used herein, the terminology "graft copolymer" means a polymer having two or more polymeric blocks connected to a polymer backbone as side chains.

As used herein in reference to molecule or a portion of a molecule, the term "polymer" means a chain of two or more repeating constitutional units ("monomeric units").

The graft copolymer comprises one or more blocks of a polymer capable of bonding to human hair grafted to a polysaccharide polymer backbone.

The polysaccharide polymer contains repeated saccharide units, and includes, for example, polygalactomannans such as guar, xanthans, polyfructoses such as levan, starches, starch derivatives, such as amylopectin, cellulose, and cellulose derivatives, such as methylcellulose, ethylcellulose, carboxymethylcellulose, hydroxyethylcellulose, cellulose acetate, cellulose acetate butyrate, and cellulose acetate propionate.

Polygalactomannans are polysaccharides consisting mainly of the monosaccharides mannose and galactose. The mannose-elements form a chain consisting of many hundreds of (1,4)-ß-D-mannopyranosyl-residues, with 1,6 linked -D-galactopyranosyl-residues at varying distances, dependent on the plant of origin. Naturally occurring galactomannans are available from numerous sources, including guar gum, guar splits, locust bean gum and tara gum. Additionally, galactomannans may also be obtained by classical synthetic routes or may be obtained by chemical modification of naturally occurring galactomannans.

Guar gum, often called "guar flour" after grinding, refers to the mucilage found in the seed of the leguminous plant *Cyamopsis tetragonolobus*. The water soluble fraction (85%) is called "guaran," which consists of linear chains of (1,4)-.beta.-D mannopyranosyl units-with α D-galactopyranosyl units attached by (1,6) linkages. The ratio of D-galactose to D-mannose in guaran is about 1:2. Guar gum may take the form of a whitish powder which is dispersible in hot or cold water. Native guar gum typically has a weight average molecular weight of between 2,000,000 and 5,000,000 Daltons (wherein 1 Dalton = 1 gram per mole ('g/mol"). Guar gum may be obtained, for example, from Rhodia, Inc. (Cranbury, New Jersey), Hercules, Inc. (Wilmington, Delaware) and TIC Gum, Inc. (Belcamp, Maryland).

Guar seeds are composed of a pair of tough, non-brittle endosperm sections, hereafter referred to as "guar splits," between which is sandwiched the brittle embryo (germ). After dehulling, the seeds are split, the germ (43-47% of the seed) is removed by screening, and the splits are ground. The ground splits are reported to contain about 78-82% galactomannan polysaccharide and minor amounts of some proteinaceous material, inorganic salts, water-insoluble gum, and cell membranes, as well as some residual seedcoat and embryo.

Locust bean gum or carob bean gum is the refined endosperm of the seed of the carob tree, *Ceratonia siliqua*. The ratio of galactose to mannose for this type of gum is about 1:4. Locust bean gum is commercially available and may be obtained, for example, from TIC Gum, Inc. (Bekamp, Maryland) and Rhodia, Inc. (Cranbury, New Jersey).

Tara gum is derived from the refined seed gum of the tara tree. The ratio of galactose to mannose is about 1:3. Tara gum is not produced in the United States commercially, but the gum may be obtained from various sources outside the United States.

Other galactomannans of interest are the modified galactomannans, including carboxymethyl guar, carboxymethylhydroxypropyl guar, cationic hydroxpropyl guar, hydroxyalkyl guar, including hydroxyethyl guar, hydroxypropyl guar, hydroxybutyl guar and higher hydroxylalkyl guars, carboxylalkyl guars, including carboxymethyl guar, carboxylpropyl guar, carboxybutyl guar, and higher alkyl carboxy guars, the hydroxyethylated, hydroxypropylated and carboxymethylated derivative of guaran, the hydroxethylated and carboxymethylated derivatives of carubin and the hydroxypropylated and carboxymethylated derivatives of cassia-gum.

Xanthans of interest are xanthan gum and xanthan gel. Xanthan gum is a polysaccharide gum produced by *Xathomonas campestris* and contains D-glucose, D-mannose, D-glucuronic acid as the main hexose units, also contains pyruvate acid, and is partially acetylated.

Levan is a polyfructose comprising 5-membered rings linked through β-2,6 bonds, with branching through β-2,1 bonds. Levan exhibits a glass transition temperature of 138°C and is available in particulate form. At a molecular weight of 1-2 million, the diameter of the densely-packed spherulitic particles is about 85 nm.

Modified celluloses are celluloses containing at least one functional group, such as a hydroxy group, hydroxycarboxyl group, or hydroxyalkyl group, such as for example, hydroxymethyl-, hydroxyethyl-, hydroxypropyl- or hydroxybutyl.

In one embodiment, the grafted blocks of the graft copolymer are derived from at least one polymer that is capable of bonding with, in the sense of forming a polar attraction to, human hair. Polymers capable of such bonding include cationic polymers, the cationic sites of which are capable of bonding with electronegative sites on the hair, as well as substantially neutral polymers that are capable of forming hydrogen bonds with hair. Suitable substantially neutral polymers that comprise repeating units that contain a covalently bonded hydrogen atom capable of forming a hydrogen bond with an electronegative site on the hair and/or an electronegative site, such as, for example, a uriedo group, that is capable of forming a hydrogen bond with a hydrogen atom of the hair.

The polymer to be grafted comprises polymeric blocks derived from at least one polymer capable of bonding with human hair and further comprises polymeric blocks derived from one or more other polymers that are not capable of bonding with human hair, with selection of the relative amounts of the two types of polymers providing a potential approach to adjusting the affinity of the graft copolymer for hair and providing a potential approach for imparting other beneficial properties to the graft copolymer.

Suitable substantially neutral polymers that are capable of forming hydrogen bonds with hair include, for example, polyamide polymers polyurethane polymers, and polyuriedo polymers.

Suitable cationic polymers are cationic derivatives of nitrogenous natural or synthetic polymers. As used herein, "nitrogenous polymer" means a polymer comprising nitrogen atoms, either in the polymer backbone, in pendant substituent groups, or in both the polymer backbone and in pendant substituent groups. Such polymers may be homopolymers of nitrogenous monomers, such as, for example, polyalkyleneamines, polyalkyleneimines, poly(vinyl pyridine), or copolymers of such nitrogenous monomers with other copolymerizable comonomers, such as, for example poly(vinyl pyridine-acrylonitrile), poly(vinyl pyridene-methyl methacrylate). Cationic derivatives of such polymers may be formed by forming amine salts of all or a portion of the nitrogen atoms of a nitrogenous polymer, by quaternizing all or a portion of the nitrogen atoms of a nitrogenous polymer to form a quaternary ammonium salts, or by oxidizing all or a portion of the nitrogen atoms of nitrogenous polymer to form N-oxide groups. As used herein, the term "amine salt" in reference to a nitrogen atom of a nitrogenous polymer means a monomeric unit of the polymer containing the nitrogen atom, wherein such nitrogen atom is covalently bonded to from one to three organic groups and is associated with an anion. As used herein, the term "quaternary ammonium salt" in reference to a nitrogen atom of a nitrogenous polymer means a monomeric unit of the polymer, wherein such nitrogen atom is covalently bonded to four organic groups and is associated with an anion.

Exemplary cationic natural polymers include quaternized polysaccharide gums, such as quaternized guar gum, quaternized locust bean gums, quaternized Xanthan gums, quaternized dextrins, and quaternized starches. As used herein, the term "quaternized" in reference to a compound means that the compound has at least quaternary ammonium salt group per molecule.

In one embodiment, the cationic synthetic polymer is an addition polymer comprising units derived from nitrogenous ethylenically unsaturated monomers, optionally in combination with non-nitrogenous ethylenically unsaturated comonomers, wherein the polymer is quaternized by, for example, by modifying a nitrogenous polymer by replacing a hydrogen atom attached to a nitrogen atom of the nitrogenous polymer with an organic group, such as, for example, an alkyl group, a dialkyl sulfate group, a poly(alkyleneoxy) group, or a carboxyl group.

In one embodiment, the cationic synthetic polymer is a condensation polymer made by condensation of a tertiary or secondary hydroxyalkylamine, for example, tri(hydroxyethyl)amine, or methyl-di(hydroxyethyl)amine, or a di(hydroxyalkyl) functional quaternary ammonium compound, such as dimethyl-di(hydroxyethyl)ammonium chloride, or a dialkylenetriamine, such as diethylenetriamine, with a dicarboxylic acid, for example, succinic acid, maleic acid, or phthalic acid, or the corresponding acid halide or anhydride, wherein the resultant polymer is rendered cationic by reaction of all or some of the nitrogen atoms of the polymer backbone with an alkyl halide or dialkyl sulphate.

In one embodiment, the cationic synthetic polymer comprises a polyamine wherein the nitrogen atoms of the polyamine backbone are totally or partially quaternized, totally or partially oxidized to N-oxides, or a combination thereof.

In another embodiment, the cationic synthetic polymer comprises a cationic polyester polymer formed by condensation of a dicarboxylic aid and a triol or higher polyhydric alcohol, and reaction some or all of the remaining hydroxyl groups of the resulting polyester with cationic groups or with nitrogenous groups that are subsequently totally or partially quaternized, totally or partially oxidized to N-oxides, or a combination thereof.

In one embodiment, the cationic polymer is a polymeric quaternary ammonium salt. Exemplary polymeric quaternary ammonium salts are disclosed in "CTFA International Cosmetic Ingredient Dictionary", Fourth Edition, J. M. Nikitakis, et al, Editors, published by the Cosmetic, Toiletry, and Fragrance Association, 1991, and include: Polyquaternium-1 (CAS Number: 68518-54-7), Polyquaternium-2 (CAS Number: 63451-274), Polyquaternium-4 (copolymer of hydroxymethyl cellulose and diallyl ammonium chloride), Polyquaternium-5 (CAS Number: 26006-224), Polyquaternium-6 (CAS Number: 26062-79-3), Polyquaternium-7 (CAS Number: 26590-05-6), Polyquaterium-9 (polymeric quaternary ammonium salt formed by quatemizing polydimethylaminoethyl methacrylate with methyl bromide), Polyquaternium-10 (polymeric quaternary ammonium salt of hydroxyethyl cellulose reacted with a trimethyl ammonium substituted epoxide), Polyquaternium-12 (CAS Number: 68877-50-9), Polyquaternium-13 (CAS Number: 68877-47-4), Polyquaternium-14 (CAS Number: 27103-90-8), Polyquaternium-15 (CAS Number: 35429-19-7), Polyquaternium-16 (polymeric quaternary ammonium salt formed from methylvinylimidazolium chloride and vinylpyrrolidone), Polyquaternium-17 (polymeric quaternary salt prepared by the reaction of adipic acid and dimethylaminopropylamine, reacted with dichloroethyl ether), Polyquaternium-18 (polymeric quaternary salt prepared by the reaction of azelaic acid and dimethylaminopropylamine reacted with dichloroethyl ether), Polyquaternium-19 (polymeric quaternary ammonium salt prepared by the reaction of polyvinyl alcohol with 2,3-epoxypropylamine), Polyquaternium-20 (polymeric quaternary ammonium salt prepared by the reaction of polyvinyl octadecyl ether with 2,3-epoxypropylamine), Polyquaternium-22 (copolymer of dimethyldiallyl ammonium chloride and acrylic acid), Polyquaternium-24 (polymeric quaternary ammonium salt of hydroxyethyl cellulose reacted with a lauryl dimethyl ammonium substituted epoxide), Polyquaternium-27 (block copolymer formed by the reaction of Polyquaternium-2 with Polyquaternium-17), Polyquaternium-28 (polymeric quaternary ammonium salt consisting of vinylpyrrolidone and dimethylaminopropyl methacrylamide monomers), Polyquaternium-29 (chitosan that has been reacted with propylene oxide and quaternized with epichlorohydrin), Polyquaternium-39 (polymeric quaternary ammonium salt of acrylic acid, diallyl dimethyl ammonium chloride, and acrylamide), Polyquaternium-43 (copolymer of acrylamide, acryamidopropyltrimonium chloride, 2-amidopropylacrylamide sulfonate, and dimethylaminopropyl methacrylate ("DMAPMA") monomers), Polyquaternium-44 (polymeric quaternary ammonium salt consisting of repeating units derived from vinyl pyrrolidone and quaternized imidazoline monomers), Polyquaternium-46 (polymeric quaternary ammonium salt formed by polymerization of vinylcaprolactam and vinyl pyrrolidone with methylvinylimidazolium), Polyquaternium-47 (polymeric quaternary ammonium chloride formed by the polymerization of acrylic acid, methyl acrylate and methacrylamidopropyltrimonium chloride), Polyquatenium-48 (copolymer of methacryloyl ethyl betaine, 2-hydroxyethyl methacrylate and methacryloyl ethyl trimethyl ammonium chloride), Polyquarernium-49 (copolymer of methacryloyl ethyl betaine, PEG-9 methacrylate and methacryloyl ethyl trimethyl ammonium chloride).

As an alternative to grafting a cationic polymer with the polysaccharide polymer, a precursor polymer may be grafted to the polysaccharide polymer and the grafted precursor polymer sections of the resulting graft copolymer subsequently rendered cationic by, for example, any of the techniques discussed above. For example, a non-quaternized nitrogenous polymer may be grafted to the polysaccharide polymer and some or all of the nitrogen atoms of the grafted nitrogenous polymer sections of the resulting graft copolymer subsequently quaternized.

In one embodiment, the cationic polymer has a molecular weight of from about 600 to about 1,000,000 g/mol, more typically from about 600 to about 500,000 g/mol, even more typically from about 1000 to 50,000 g/mol. In one embodiment, the cationic polymer is at least substantially, that is, at least 0.5% polymer by weight at 20 °C, soluble in water. Suitable cationic polymers typically exhibit a charge density of 0.01 to about 2 milli-equivalents per gram ("meq/g"), more typically from about 0.05 to about 1 meq/g and even more typically from about 0.1 to about 0.2 meq/g.

Alternatively, the graft copolymer of the present invention may be synthesized by irradiating a mixture of the polysaccharide polymer and an unsaturated monomer, typically an ethylenically unsaturated monomer and then rendering the resulting graft copolymer cationic by, for example, any of the techniques discussed above. The monomer to be grafted may be a single monomer or a mixture of monomers.

In one embodiment, the monomer to be grafted includes at least one monomer that contains at least one ethylenically unsaturated site per molecule of monomer and at least one nitrogenous functional group per molecule of monomer, such as, for example, methacrylamidopropyl trimethylammonium chloride, acrylamidodimethyl propane sulfuric acid, vinyl pyridine, vinyl pyridinium halides, vinyl pyrrolidone, vinyl caprolactam, N-alkylated acrylamide, and (N,N-dimethyl amino)-2-ethyl methacrylate.

The polysaccharide and the monomer or polymer capable of bonding to human hair are grafted together by chemical reaction of the polysaccharide and the monomer or polymer. In one embodiment, the reaction is initiated by exposure of a mixture of the polysaccharide and the monomer or polymer to ionizing radiation, such as, for example, electron beam radiation. Alternatively, the reaction may be initiated by a chemical initiator, such as, for example, organic peroxides, or hydroperoxides or azo-derivatives, such as benzoyl peroxide, cumene peroxide, ter-butyl-perbenzoate, and azo-bis-isobutyronitrile.

Irradiation with electron beam radiation may cause a controllable depolymerization of the polysaccharide polymer. The degree of depolymerization obtained in carrying out the method of the invention is influenced by the molecular weight of the original polymer being treated and the desired molecular weight of the depolymerized product. Guar gum has a molecular weight of over 2,000,000 Daltons and generally between 2,000,000 Daltons and 5,000,000 Daltons. In the normal operation of method of this invention, the polysaccharide polymer will be depolymerized to a pre-selected value, typically to below about 700,000 Daltons, more typically to below about 500,000 Daltons and even more preferably to below about 300,000 Daltons.

The polysaccharide polymer is typically reacted in the solid phase. The term "solid phase" includes powders, granules, flakes, particles, and the like.

Initially, the polysaccharide and monomer or polymer to be grafted are mixed together. The irradiation is applied directly to the mixture of polysaccharide and monomer or polymer in the solid phase, preferably as the polymer passes on trays on a production line continuous belt. The polysaccharide to be modified, with or without depolymerization, is placed in a container, for example, a tray, up to a thickness that allows complete penetration of the solid material by the high energy electron beams. The polydispersity of the graft copolymer product is reduced if all of the reactant material is penetrated by the electron beam, so the layer of solid material exposed to the high energy electron beam should have a substantially uniform thickness if a low polydispersity is desired. The mixture may, optionally, be covered with a radiation pervious thin plastic film for safety purposes. The mixture is then irradiated with high energy electron beams at a specified dose rate, depending upon the extent of modification of the polymer to be obtained. Irradiation is typically accomplished by carrying the container of the mixture, for example, by a conveyor, through a radiation chamber.

In irradiation processing, dose is defined as the amount of energy absorbed by the target material. Dosages are defined either in units of grays or mega rads. One kilogray is equal to 1,000 joules per kilogram. A mega rad is equal to 1,000,000 ergs per gram. Accordingly, one mega rad equals ten kilogray. A typical dose of radiation is between about 0.5 and about 20 mega rads or about 5 to about 200 kilogray (kGy), which can be generated by a 4.5 MeV generator operating at 15 milliamps. Such generators are available from E-Beam Services, Inc., of Plainview, New York.

The dose rate is the amount of time required to provide the irradiation dosage needed to graft the monomer or polymer to the polysaccharide and, optionally, to depolymerize the polymer to the selected molecular weight. This rate has a direct bearing on the length of time it takes to deliver a given dose, and therefore the amount of time that the polymer is exposed to ionizing radiation. High power electron beams generate high irradiation doses rapidly. The use of high power beams allows a much higher production rate of graft copolymer product. The high voltage electron beam irradiation of the polymer is carried out preferably at room temperature.

Typically, a high energy electron beam generator of 1-10 MeV is used for practicing this invention because it penetrates deep into the materials, allowing a thicker layer of material to be irradiated. Higher than 10 MeV may be used, but may undesirably generate radioactivity from high-Z elements. A high voltage electron beam generator may be obtained from Electron Solutions Inc. and Science Research Laboratory, Somerville, Massachusetts, Ion Beam Applications, Louvain-la-Neuve, Belgium, and The Titan Corporation, San Diego, California.

A low energy electron beam generator (150 kV - 1 MeV) can also be used. The material will be irradiated as a layer as it passes through the beam; optionally, the irradiation is performed after the material has been mechanically ground to a powder. Such a generator is generally cheaper and does not require concrete shielding. A low voltage electron beam generator may be obtained from EZCure by Energy Sciences, Inc., Wilmington, Massachusetts, Easy E-beam by Radiation Dynamics Inc., Edgewood, New York and EB-ATP by Electron Solutions Inc., Somerville, Massachusetts. This equipment is conventionally used primarily for surface irradiation curing. For example, electron beams having low-energy (1.3 MeV) and high-power (> 100 kW) can be used for polysaccharide grafting/depolymerization. The 100 kW power rating would be capable of grafting/depolymerizing 2400 kg/hour at a dose of 15 Mrad or 12,000 kg/hour at a dose of 3 Mrad.

In one embodiment, the graft copolymer is water soluble or water dispersible.

Suitable carriers for the personal care composition of the present invention are liquid or solid vehicles that is capable of being mixed with graft polymer of the present invention for delivery of the polymer to the hair and that will not cause harm when topically applied to the hair or skin. Suitable liquid carriers include water, organic solvents, such as alkanols, acetone, and isoparaffins, and alkylether diols, mixtures of such organic solvents, and mixtures of water with water miscible organic solvents. Suitable alkanols are typically (C₁-C₁₈)alkanols and include monohydric alkanols, such as methanol, ethanol, isopropanol, Cetyl Alcohol, Stearyl Alcohol, Cetearyl Alcohol, Benzyl Alcohol, Oleyl Alcohol, and polyhydric alkanols, such as 2-butoxyethanol, and ethylene glycol, as well as mixtures of such alkanols. Suitable alkylether diols include, for example, ethylene glycol monoethyl ether, propylene glycol monoethyl ether and diethylene glycol monomethyl ether, and mixtures thereof.

In one embodiment, the personal care composition according the present invention is an aqueous system wherein the carrier comprises water.

Hair care compositions include, for example, shampoos, conditioners, and styling products such as, for example, styling creams, gel, and mousses.

In one embodiment, the hair care composition of the present invention comprises, based on 100 parts by weight (pbw") of such composition, from about 0.01 pbw to about 10 pbw, more typically from about 0.05 pbw to about 5 pbw, of the graft copolymer of the present invention and from about 90 to about 99.99 pbw carrier, more typically from about 95 to about 99.95 pbw of a carrier.

In one embodiment, the personal care composition is a hair styling composition that is in the form of a gel, mousse, or spray and may be applied to the hair, for example, by hand or by spraying, as appropriate in view of the form of the composition.

In one embodiment, the personal care composition is a hair styling gel that comprises a graft copolymer according to the-present invention, a carrier selected from water, (C₂-C₆)alkanols, and mixtures thereof, and, optionally, a thickener. Suitable thickeners include acrylic acid polymers and copolymers, such as a Carbomer.

As used herein, the term "mousse" means a composition that is in the form of a foam when applied. In one embodiment, the personal care composition is a hair styling mousse is packaged in a pressurized container and comprises a graft copolymer according to the present invention, a carrier, and a propellant suitable for foaming the composition when the composition is dispensed from the container. Suitable propellants are liquefiable gases, such as, for example, propane, butane, isobutane, nitrogen, carbon dioxide, nitrous oxide, 1,2-difluoroethane.

In one embodiment, the personal care composition is a hair spray composition suitable for spray application from a container that is equipped with a mechanical sprayer, comprising a graft copolymer according to the present invention and a carrier selected from water, (C₂-C₆)alkanols, and mixtures thereof.

In one embodiment, the personal care composition is an aerosol hair spray composition suitable for spray application from a pressurized container and comprises, a graft copolymer according to the present invention, a carrier, typically a (C₁-C₆)alkanol or (C₇-C₁₀) isoparaffin carrier, and a propellant suitable for aerosol delivery of the hair spray composition to the hair. Suitable propellants are those described above in regard to the hair styling mousse embodiment of the personal care composition of the present invention.

In one embodiment, the hair care composition of the present invention is a shampoo composition or a conditioning composition and contains one or more surfactant compounds. Surfactant compounds are characterized by the presence of both a hydrophilic group and a hydrophobic group on the same molecule and include amphoteric surfactants, Zwitterionic surfactants, nonionic surfactants, anionic surfactants, cationic surfactants or combinations thereof.

In one embodiment, the hair care composition of the present invention comprises, based on 100 pbw of such composition, from about 0.01 pbw to about 10 pbw, more typically from about 0.05 pbw to about 5 pbw, of the graft copolymer of the present invention, from about 0.01 to about 25 pbw, more typically from about 0.05 to about 15 pbw, of one or more surfactants and from about 65 to about 99.98 pbw carrier, more typically from about 80 to about 99 9 pbw, of a carrier.

Anionic surfactants are ionic surfactant compounds that have a negative electrical charge associated with the hydrophilic portion of the surfactant. Any anionic surfactant that is acceptable for use in the intended end use application is suitable as the anionic surfactant component of the composition of the present invention. Examples of suitable anionic surfactants include, generally, alkyl sulfonates, aryl sulfonates, alkaryl sulfonates, alpha olefin sulfonates, paraffin sulfonates, alkyl ester sulfonates, alkyl sulfates, alkyl alkoxy sulfates, alkyl alkoxy carboxylates, alkyl alkoxylated sulfates, acylsarcosinates, and amidosulfonates, as well as mixtures thereof. Specific examples of suitable anionic surfactants include sodium tridecyl benzene sulfonate, sodium dodecyl benzene sulfonate, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, ammonium cocyl sulfate, ammonium lauroyl sulfate, sodium cocyl sulfate, sodium lauroyl sulfate, potassium cocyl sulfate, potassium lauryl sulfate, monoethanolamine cocyl sulfate, sodium lauryl sarcosinate, sodium lauroyl sarcosinate, lauryl sarcosine, and cocyl sarcosine.

Cationic surfactants are ionic surfactant compounds that have a positive electrical charge associated with the hydrophilic portion of the surfactant. Any cationic surfactant that is acceptable for use in the intended end use application is suitable as cationic surfactant component of the composition of the present invention. Examples of suitable cationic surfactants include compounds according to formula (I) below: 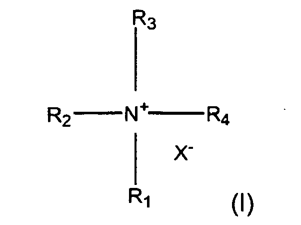 wherein:
R₁, R₂, R₃ and R₄, are each independently hydrogen, an organic group, provided that at least one of R₁, R₂, R₃ and R₄ is not hydrogen.
X⁻ is an anion.

Suitable anions include, for example, chloride, bromide, methosulfate, ethosulfate, lactate, saccharinate, acetate or phosphate.

If one to three of the R₁, R₂, R₃ and R₄ groups are hydrogen, then the compound may be referred to as an amine salt. Some examples of cationic amine salts include polyethoxylated (2) oleyl/stearyl amine, ethoxylated tallow amine, cocoalkylamine, oleylamine, and tallow alkyl amine.

For quaternary ammonium compounds, R_{1,} R_{2,} R_{3,} and R₄ may each independently be the same or different organic group, or alternatively, may be fused with another one of the R₁, R₂, R₃ and R₄ groups to form, together with the nitrogen atom to which they are attached, a heterocyclic ring, but may not be hydrogen. Suitable organic groups include, for example, alkyl, alkoxy, hydroxyalkyl, and aryl, each of which may be further substituted with other organic groups. Suitable quaternary ammonium compounds include monoalkyl amine derivatives, dialkyl amine derivatives, and imidazoline derivatives.

Suitable monoalkyl amine derivatives include, for example, cetyl trimethyl ammonium bromide (also known as CETAB or cetrimonium bromide), cetyl trimethyl ammonium chloride (also known as cetrimonium chloride), myristyl trimethyl ammonium bromide (also known as myrtrimonium bromide or Quaternium-13), stearyl dimethyl benzyl ammonium chloride (also known as stearalkonium chloride), oleyl dimethyl benzyl ammonium chloride, (also known as olealkonium chloride), lauryl/myristryl trimethyl ammonium methosulfate (also known as cocotrimonium methosulfate), cetyl-dimethyl-(2)hydroxyethyl ammonium dihydrogen phosphate (also known as hydroxyethyl cetyldimonium phosphate), bassuamidopropylkonium chloride, cocotrimonium chloride, distearyldimonium chloride, wheat germ-amidopropalkonium chloride, stearyl octyldimonium methosulfate, isostearaminopropal-konium chloride, dihydroxypropyl PEG-5 linoleaminium chloride, PEG-2 stearmonium chloride, Quaternium 18, Quaternium 80, Quaternium 82, Quaternium 84, behentrimonium chloride, dicetyl dimonium chloride, behentrimonium methosulfate, tallow trimonium chloride and behenamidopropyl ethyl dimonium ethosulfate.

Suitable dialkyl amine derivatives include, for example, distearyldimonium chloride, dicetyl dimonium chloride, stearyl octyldimonium methosulfate, dihydrogenated palmoylethyl hydroxyethylmonium methosulfate, dipalmitoylethyl hydroxyethylmonium methosulfate, dioleoylethyl hydroxyethylmonium methosulfate, hydroxypropyl bisstearyldimonium chloride and mixtures thereof.

Suitable imidazoline derivatives include, for example, isostearyl benzylimidonium chloride, cocoyl benzyl hydroxyethyl imidazolinium chloride, cocoyl hydroxyethylimidazolinium PG-chloride phosphate, Quaternium 32, and stearyl hydroxyethylimidonium chloride, and mixtures thereof.

Amphoteric surfactants are ionic surfactant compounds that are characterized by the presence of two ionic sites on the same molecule and which, depending on the pH of the surrounding medium, may carry a negative electrical charge, a positive electrical charge, or both a negative electrical charge and a positive electrical charge on the same molecule. Any amphoteric surfactant that is acceptable for use in the intended end use application is suitable as the optional amphoteric surfactant component of the composition of the present invention. Examples of suitable amphoteric surfactants include derivatives of aliphatic secondary and tertiary amines in which the aliphatic radical can be straight chain or branched and wherein one of the aliphatic substituents contains from about 8 to about 18 carbon atoms and one contains an anionic water solubilizing group. Specific examples of suitable amphoteric surfactants include the alkali metal, alkaline earth metal, ammonium or substituted ammonium salts of alkyl amphocarboxy glycinates and alkyl amphocarboxypropionates, alkyl amphodipropionates, alkyl amphodiacetates, alkyl amphoglycinates and alkyl amphopropionates, as well as alkyl iminopropionates, alkyl iminodipropionates and alkyl amphopropylsulfonates, such as for example, cocoamphoacetate cocoamphopropionate, cocoamphodiacetate, lauroamphoacetate, lauroamphodiacetate , lauroamphodipropionate, lauroamphodiacetate, cocoamphopropyl sulfonate caproamphodiacetate, caproamphoacetate, caproamphodipropionate, and stearoamphoacetate.

Zwitterionic surfactants are ionic surfactant compounds characterized by the presence of two ionic sites per molecule, wherein one of the ionic sites carries a positive electrical charge regardless of the pH of the surrounding medium and wherein the other ionic site may, depending on the pH of the surrounding medium, carry a positive charge. Any Zwitterionic surfactant that is acceptable for use in the intended end use application is suitable as the optional Zwitterionic surfactant component of the composition of the present invention. Examples of suitable Zwitterionic surfactants include those which can be broadly described as derivatives of aliphatic quaternary ammonium, phosphonium, and sulfonium compounds in which the aliphatic radicals can be straight chain or branched and wherein one of the aliphatic substituents contains from about 8 to 18 carbon atoms and one contains an anionic water-solubilizing group such as carboxyl, sulfonate, sulfate, phosphate or phosphonate. Specific examples of suitable Zwitterionic surfactants include alkyl betaines, such as cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine and alkylamidopropylhydroxy sultaines.

Nonionic surfactants are surfactant compounds that do not dissociate into ions and that not have an electrical charge associated with them. Any nonionic surfactant that is acceptable for use in the intended end use application is suitable as the optional nonionic surfactant component of the composition of the present invention. Examples of suitable nonionic surfactants include compounds produced by the condensation of alkylene oxide groups (hydrophilic in nature) with an organic hydrophobic compound which may be aliphatic or alkyl aromatic in nature. Examples of useful nonionic surfactants include the polyethylene, polypropylene, and polybutylene oxide condensates of alkyl phenols, fatty acid amide surfactants, polyhydroxy fatty acid amide surfactants, amine oxide surfactants, alkyl ethoxylate surfactants, alkanoyl glucose amide surfactants, alkanolamides surfactants, alkylpolyglycosides, and condensation products of aliphatic alcohols with from about 1 to about 25 moles of ethylene oxide. Specific examples of suitable nonionic surfactants include alkanolamides such as cocamide DEA, cocamide MEA, cocamide MIPA, PEG-5 cocamide MEA, lauramide DEA, and lauramide MEA; alkyl amine oxides such as lauramine oxide, cocamine oxide, cocamidopropylamine oxide, and lauramidopropylamine oxide; polysorbates and ethoxylated sorbitan esters such as sorbitan laurate, sorbitan distearate, PEG-80 sorbitan laurate, polysorbate-20, and polysorbate-80; fatty acids or fatty acid esters such as lauric acid, isostearic acid, and PEG-150 distearate; fatty alcohols or ethoxylated fatty alcohols such as lauryl alcohol, laureth-4, laureth-7, laureth-9, laureth-40, trideceth alcohol, C11-15 pareth-9, C12-13 Pareth-3, and C14-15 Pareth-11.

Hair care compositions according to the present invention may, optionally, further contain other ingredients, such as, for example, preservatives such as benzyl alcohol, methyl paraben, propyl paraben and imidazolidinyl urea, thickeners and viscosity modifiers such as block polymers of ethylene oxide and propylene oxide, electrolytes, such as sodium chloride, sodium sulfate, polyvinyl alcohol, and ethyl alcohol, pH adjusting agents such as citric acid, succinic acid, phosphoric acid, sodium hydroxide, sodium carbonate, perfumes, dyes, other conditioning agents such as organosilicon materials, including, silicone gums, polyorganosiloxane fluids, and silicone resins, that is, crosslinked polyorganosiloxane systems, active ingredients such as anti-dandruff agents (zinc pyrithion), vitamins or their derivatives such as Vitamin B, Vitamin E Acetate, and sequestering agents such as disodium ethylene diamine tetra-acetate.

In one embodiment, the hair care composition of the present invention comprises, based on 100 pbw of the hair care composition and independently for each such ingredient, up to about 10 pbw, preferably from 0.5 pbw to about 5.0 pbw, of such other ingredients, depending on the desired properties of the hair care composition.

The personal care composition of the present invention is used by applying the personal composition to the hair.

### Example 1 (Comparative)

Hydroxyethylcellulose, (Cellosize HEC QP 100M-H, Dow) was sprayed in a mixer with methacrylamidopropyl trimethylammonium chloride ("MAPTAC", in the form of a 50% aqueous solution, Degussa) at the weight ratios of the active components shown in Table I, and then thoroughly mixed. The MAPTAC-swelled cellulose was then air-dried to a moisture contents of 3 - 15 percent by weight and ground into a powder. The ground samples were then packed in individual plastic bags to provide a sample thickeness of less than about 3 cm. The bags were placed on a tray, which was carried by a conveyor into a radiation chamber. The samples were irradiated at the given dose by electron beam generated by a 4.5 MeV generator above the top surface of the tray. The desired dose was obtained by adjusting the linear velocity of the conveyor and/or the beam current. To further reduce the residual amount of MAPTAC, the irradiated samples were then sprayed with 1 part of 10% sodium metabisufite in 1:1 water/methanol solution per 50 part by weight of the irradiated samples, and then cured in vacuum oven at 65 - 70 ° for 2 hours. The residual MAPTAC in the samples was measured by HPLC analysis after the irradiation (Table I) and after the post cure treatment (Table II).

The molecular weight of the irradiated and post cured samples was analyzed by gel permeation chromatogram (column, Supelco Progel-TSK G3000PW_{XL} and G6000PW_{XL} in series; mobile phase, 55 mM Na₂SO₄, 0.02% NaN₃; flow rate, 0.6 ml/min; detector, Waters 410 Refractive Index; inj. Volume, 200 µl; temperature, 40° C). The samples were dissolved in the mobile phase to give 0.025% solutions by weight. The calibration curve was generated using stachyose and two guar samples with molecular weights of 667 Daltons, 58,000 Daltons and 2,000,000 Daltons. The weight average molecular weight is shown in Table III. Little or no homopolymer of MAPTAC was detected by the GPC analysis. A distinct peak would be observed at lower molecular part of the chromatogram if there is any homopolymer. The grafted polymer can also be isolated from aqueous methanol solution by precipitating with acetone.

**Table I. Percentage of MAPTAC in Sample after Irradiation**

| Dosage (MRad) | 0.5 | 1 | 3 | 5 | 10 | 20 |
|---|---|---|---|---|---|---|
| HEC/MAPTAC: | | | | | | |
| 85/15 | 2.18 | 0.76 | 1.66 | 0.060 | 0.044 | 0.042 |
| 70/30 | 1.96 | 0.71 | 0.138 | 0.10 | 0.067 | 0.13 |
| 50/50 | 2.23 | 1.58 | 0.19 | 0.21 | 0.14 | 0.11 |

**Table II. Percentage of MAPTAC in Sample after Post-cure treatment**

| Dosage (MRad) | 0.5 | 1 | 3 | 5 | 10 | 20 |
|---|---|---|---|---|---|---|
| HEC/MAPTAC: | | | | | | |
| 85/15 | 0.13 | 0.033 | 0.012 | -- | -- | 0.0027 |
| 70/30 | 0.021 | -- | -- | -- | -- | -- |
| 50/50 | 0.0113 | -- | -- | -- | -- | -- |

| | | | | | | |
|---|---|---|---|---|---|---|
| ND = not detectable | | | | | | |

**Table III. GPC Molecular Weight ("M_{w}") for Selected Samples**

| Dosage (MRad) | 0.5 | 1 | 3 | 5 | 10 | 20 |
|---|---|---|---|---|---|---|
| HEC/MAPTAC: | 552,045 | 336,938 | 283,000 | 227,108 | 119,427 | 61,761 |
| 85/15 | | | | | | |
| 70/30 | 488,034 | 253,090 | -- | 189,296 | 100,519 | 56,290 |
| 50/50 | 309,633 | 302,508 | -- | 187,132 | 102,965 | 67,974 |

An almost quatitative conversion was obtained with a grafting rate of MAPTAC greater than 85% for all samples.

### Example 2 (Comparative)

Hydroxypropylguar (Jaguar 8000, HP 140 or 105, Rhodia, Cranbury, NJ) was sprayed with MAPTAC (50% in water from Degussa) according to the ratios listed in Table IV, then throughly mixed. The wet mixture was then dried in vacuum at 50 - 70°C to the moisture content specified in Table IV. The samples were irradiated as described above in Example 1. The residual amount of non-reacted MAPTAC was analyzed by HPLC to determine the conversion of MAPTAC, that is, the percetange of MAPTAC converted into polymer. and weight average molecluar weights of the grafted polymers were measured by GPC as described above in Example 1. Selected samples were purified by Soxhlet extraction of MAPTAC homopolymer with methanol, and then, after the polysaccharide was digested with trifluoroacetic acid, analyzed for grafting rates by proton NMR.

The results set forth below in Table IV demonstrate that higher conversion only can be obtained consistently at moisture contents between 8 and 25%. Almost quantitative grafting rate was observed at moisture contend higher than 13%. Less depolymerization was observed with increasing moisture content.

**Table IV. Effect of Moisture on the Irradiation Grafting of Jaguar HP 140 and HP 105 (3 Mrad surface dose was applied)**

| 4/1 Jaguar HP140/MAPTAC | | | | 7/3 Jaguar HP105/MAPTAC | |
|---|---|---|---|---|---|
| % H₂O | %Conversion | %MAPTAC grafted | M_{w} | % H₂O | %Conversion |
| 2.6 | 82.3 | 33 | 187,000 | 5 | 52.24 |
| 7.8 | 86.9 | 31 | 171,000 | 10 | 97.72 |
| 8.8 | 99.2 | 76 | 235,000 | 15 | 98.77 |
| 13.2 | 99.7 | 97 | 267,000 | 20 | 99.20 |
| | | | | 25 | 96.17 |
| | | | | 30 | 72.99 |

The conversion of MAPTAC increased with increasing irradiation doses, while the molecular weights decreased. Even at low dose, good conversion was obtained at favorable moisture content (Table V).

**Table V. Effect of Dose on the Irradiation Grafting of Jaguar HP 140 and 105**

| Dose | 4/1 Jaguar HP140/MAPTAC at 2.6% water | | 7/3 Jaguar HP105/MAPTAC at 10% water |
|---|---|---|---|
| | %Conversion | M_{w} | %Conversion |
| 0.5 | 54.2 | 463,000 | 82.8 |
| 1.0 | 65.4 | 328,000 | 94.9 |
| 1.5 | 70.4 | 272,000 | -- |
| 2.0 | 73.2 | 241,000 | -- |
| 2.5 | 78.3 | 209,000 | -- |
| 3.0 | 82.3 | 187,000 | 97.2 |
| 5.0 | -- | -- | 99.4 |
| 10.0 | -- | -- | 99.9 |
| 15.0 | -- | -- | 99.9 |

### Comparative Examples 3 and 4 and Comparative Example 5

The shampoo composition of Comparative Example 3 was made by combining, based on 100 pbw of shampoo composition, 0.3 pbw of graft copolymer (50 wt% HEC /50 wt% MAPTAC) made according to the procedure described in Comparative Example 1, 14 pbw of an anionic surfactant (sodium laureth sulfate), 2 pbw of a Zwitterionic surfactant (cocamidopropyl betaine), 1.6 pbw of an electrolyte (NaCl), 0.05 pbw of a biocide (Kathon CG) and the remainder deionized water and adjusting the pH with citric acid and/or sodium hydroxide to within the range 6.0 to 6.5.

The shampoo composition of Comparative Example 4 was made by combining, based on 100 pbw of shampoo composition, 0.3 pbw of graft copolymer (50 wt% HEC /50 wt% MAPTAC) made according to the procedure described in Comparative Example 1, 38.5 pbw of a surfactant blend (containing water, PEG-80 sorbitan laurate, cocamidopropyl betaine, sodium trideceth sulfate, glycerin, sodium lauroamphoacetate, PEG-150 distearate, sodium laureth-13 carboxylate, citric acid, quaternium-15, and tetrasodium EDTA, Miracare® BC27, Rhodia Inc.), and the remainder deionized water, and adjusting the pH with citric acid / sodium hydroxide to within the range 6.0 to 7.0.

The shampoo composition of Comparative Example 5 was made by substituting guar hydroxypropyl trimonium chloride (Jaguar C17, (Rhodia Inc.)) for the graft copolymer in a shampoo composition otherwise directly analogous to the composition of Comparative Example 3.

Medium brown bleached hair tresses, each 2.0 gm in weight and measuring 2.54 cm wide by 16 cm long, were moistened with tap water, washed with 0.2 ml of the shampoo composition of Example 2 or 3 or Comparative Example 5 and rinsed. The work required to comb of the wet tresses after rinsing was measured using a Dia-Stron miniature tensile tester. Six tresses were used for each treatment condition. Results are given in TABLE VI below as mean values and the 95% Biferroni interval for each set of measurements.

**TABLE VI**

| | Ex. 3 | Ex. 4 | C. Ex. C1 |
|---|---|---|---|
| Work (Joules) | | | |
| Mean | 0.135 | 0.107 | 0.137 |
| 95% Biferroni | 0.12 to 0.148 | 0.087 to 0.127 | 0.123 to 0.150 |
| interval | | | |

The results indicate that the shampoo compositions of Comparative Examples 3 and 4, which contained the HEC/MAPTAC graft copolymer, provided wet combing and conditioning benefits comparable to those provided by the analogous shampoo composition of Comparative Example 5, which contained the Jaguar C17 conditioning polymer.

## Claims

1. A graft copolymer comprising one or more blocks of a polymer capable of bonding to human hair and blocks of one or more polymers that are not capable of bonding with human hair grafted to a polysaccharide polymer backbone, wherein the polymer capable of bonding to human hair is a cationic polymer, or a substantially neutral polymer capable of forming hydrogen bonds with hair.

2. The graft copolymer of claim 1, wherein the grafting is initiated by exposing a mixture comprising:
(a) a polysaccharide polymer, and
(b) one or more of:
(b)(i) a polymer capable of bonding to human hair, or
(b)(ii) a polymeric precursor of a polymer capable of bonding to human hair, or
(b)(iii) one or more unsaturated monomeric precursors of a polymer capable of bonding to human hair,
to ionizing radiation.

3. The graft copolymer of claim 2, wherein blocks derived from the polymeric precursor or the monomeric precursor are rendered cationic subsequent to grafting.

4. The graft copolymer of claim 2, wherein the mixture comprises one or more unsaturated monomer that contains at least one ethylenically unsaturated site per molecule of monomer and at least one nitrogenous functional group per molecule of monomer.

5. The graft copolymer of claim 2, wherein the polysaccharide polymer backbone is derived from a polymer selected from polygalactomannans, xanthans, polyfructoses, starches, starch derivatives, cellulose, cellulose derivatives, and mixtures thereof.

6. The graft copolymer of claim 1, wherein the polysaccharide polymer backbone is derived from a guar gum having a weight average molecular weight prior to grafting of from about 2,000,000 and 5,000,000 grams per mole.

7. The graft copolymer of claim 1, wherein the polymer capable of bonding to human hair comprises a cationic polymer.

8. The graft copolymer of claim 1, wherein the polymer capable of bonding to human hair comprises a substantially neutral polymer selected from polyamide polymers, polyurethane polymers, and polyuriedo polymers.

9. The graft copolymer of claim 1, wherein the polymer capable of bonding to human hair comprises a cationic polymer selected from quaternized polysaccharide gums.

10. The graft copolymer of claim 1, wherein the polymer capable of bonding to human hair comprises a cationic synthetic polymer selected from cationic addition polymers and cationic condensation polymers.

11. The graft copolymer of claim 1, wherein the polymer capable of bonding to human hair comprises a polymeric quaternary ammonium salt.

12. A method for making a graft copolymer according to claim 1; comprising:
mixing the polysaccharide polymer, the polymer capable of bonding to human hair, and the polymer not capable of bonding with human hair; and
irradiating the polymer mixture with electron beam irradiation to form the graft copolymer.

13. The method of claim 12, wherein the polymer mixture is irradiated with a radiation dose of from 0.5 and 20 mega rads.

14. A hair care composition, comprising the graft copolymer of claim 1 and a carrier.

15. The hair care composition of claim 14, wherein the carrier comprises water.

16. The hair care composition of claim 14, wherein the hair care composition is selected from shampoo compositions, hair conditioner compositions, and hair styling compositions.

17. The hair care composition of claim 14, wherein the hair care composition is a hair styling product selected from hair styling gels, hair styling mousses, and hair styling sprays.

18. The hair care composition of claim 14, wherein the hair care composition is a shampoo composition or a hair conditioner composition and further comprises one or more surfactants.

## Patentansprüche

1. Pfropfcopolymer, umfassend einen Block oder mehrere Blöcke eines Polymers, das zur Bindung an menschliches Haar fähig ist, und Blöcke eines Polymers oder mehrerer Polymere, die nicht fähig sind, an menschliches Haar zu binden, gepfropft auf eine Polysaccharidpolymerhauptkette, wobei das Polymer, das fähig ist, an menschliches Haar zu binden, ein kationisches Polymer oder ein im Wesentlichen neutrales Polymer ist, das fähig ist, Wasserstcaffbindungen mit Haar zu bilden.

2. Pfropfcopolymer gemäß Anspruch 1, wobei die Pfropfung initiiert wird, indem ein Gemisch, umfassend:
(a) ein Polysaccharidpolymer und
(b) eines oder mehrere von:
(b) (i) einem Polymer, das fähig ist, an menschliches Haar zu binden, oder
(b) (ii) einem polymeren Vorläufer eines Polymers, das fähig ist, man menschliches Haar zu binden, oder
(b) (iii) einem oder mehreren ungesättigen monomeren Vorläufer(n) eines Polymers, das fähig ist, an menschliches Haar zu binden,
ionisierender Strahlung ausgesetzt wird.

3. Pfropfcopolymer gemäß Anspruch 2, wobei Böcke, die von dem polymeren Vorläufer oder dem monomeren Vorläufer abgeleitet sind, mach Pfropfung kationisch gemacht werden.

4. Pfropfcopolymer gemäß Anspruch 2, wobei das Gemisch ein oder mehrere ungesättigte(s) Monomer(e) umfasst, das/die wenigstens eine ethylenisch ungesättigte Stelle pro Monomermolekül und wenigstens eine stickstoffhaltige funktionelle Gruppe pro Monomermolekül enthält/enthalten.

5. Pfropfcopolymer gemäß Anspruch 2, wobei die Polysaccharidpolymerhauptkette von einem Polymer, ausgewählt aus Polygalaktomannanen, Xanthinen, Polyfruktosen, Stärken, Stärkederivaten, Cellulose, Cellulosederivaten und Gemischen davor, abgeleitet ist.

6. Pfropfcopolymer gemäß Anspruch 1, wobei die Polysaccharidpolymerhauptkette von einem Guar-Gummi abgeleitet ist, das vor Pfropfung ein gewichtsmittleres Molekulargewicht von etwa 2.000.000 bis 5.000.000 Gramm pro Mol hat.

7. Pfropfcopolymer gemäß Anspruch 1, wobei das Polymer, das fähig ist, an menschliches Haar zu binden, ein kationisches Polymer umfasst.

8. Pfropfcopolymer gemäß Anspruch 1, wobei das Polymer, das fähig ist, an menschliches Haar zu binden, ein im Wesentliches neutrales Polymer umfasst, das aus Polyamidpolymeren, Polyurethanpolymeren und Polyureidopolymeren ausgewählt ist.

9. Pfropfcopolymer gemäß Anspruch 1, wobei das Polymer, das fähig ist, an menschliches Haar zu binden, ein kationisches Polymer umfasst, das aus quaternisierten Polysaccharidgummen ausgewählt ist.

10. Pfropfcopolymer gemäß Anspruch 1, wobei das Polymer, das fähig ist, an menschliches Haar zu binden, ein kationisches synthetisches Polymer umfasst, das aus kationischen Additionspolymeren und kationischen Kondensationspolymeren ausgewählt ist.

11. Pfropfcopolymer gemäß Anspruch 1, wobei das Polymer, das fähig ist, an menschliches Haar zu binden, ein polymeres quaternäres Ammoniumsalz umfasst.

12. Verfahren zur Herstellung eines Pfropfcopolymers gemäß Anspruch 1, umfassend:
Mischen des Polysaccharidpolymers, das Polymers, das fähig ist, an menschliches Haar zu binden, und des Polymers, das nicht fähig ist, an menschliches Haar zu binden, und
Bestrahlen des Polymergemisches mit Elektronenstrahlbestrahlung unter Bildung des Pfropfcopolymers.

13. Verfahren gemäß Anspruch 12, wobei das Polymergemisch mit einer Strahlungsdosis von 0,5 und 20 Mega-Rad bestrahlt wird.

14. Haarpflegezusammensetzung, umfassend das Pfropfcopolymer gemäß Anspruch 1 und einen Träger.

15. Haarpflegezusammensetzung gemäß Anspruch 14, wobei der Träger Wasser umfasst.

16. Haarpflegezusammensetzung gemäß Anspruch 14, wobei die Haarpflegezusammensetzung aus Shampoo-Zusammensetzungen, Haar-Conditioner-Zusammensetzungen und Haar-Styling-Zusammensetzungen ausgewählt ist.

17. Haarpflegezusammensetzung gemäß Anspruch 14, wobei die Haarpflegezusammensetzung ein Haar-Styling-Produkt ist, das aus Haar-Styling-Gelen, Haar-Styling-Schäumen und Haar-Styling-Sprays ausgewählt ist.

18. Haarpflegezusammensetzung gemäß Anspruch 14, wobei die Haarpflegezusammensetzung eine Shampoo-Zusammensetzung oder eine Haar-Conditioner-Zusammensetzung ist und außerdem ein oder mehrere Tensid(e) umfasst.

## Revendications

1. Copolymère greffé comprenait un ou plusieurs blocs d'un polymère susceptible de se lier à des cheveux humains et des blocs d'un ou plusieurs polymères qui ne sont pas susceptibles de se lier à des cheveux humains greffés à un squelette de polymère de polysaccharide, le polymère susceptible de se lier à des cheveux humains étant un polymère cationique ou un polymère essentiellement neutre susceptible de former des liaisons hydrogènes avec les cheveux.

2. Copolymère greffé selon la revendication 1, dans lequel le greffage est initié par exposition d'un mélange comprenant:
(a) un polymère de polysaccharide, et
(b) un ou plusieurs des composés suivants :
(b)(i) un polymère susceptible de se lier à des cheveux humains, ou
(b)(ii) un précurseur polymère d'un polymère susceptible de se lier à des cheveux humains, ou
(b)(iii) un ou plusieurs précurseurs monomères insaturés d'un polymère susceptible de se lier à des cheveux humains,
à un rayonnement ionisant.

3. Copolymère greffé selon la revendication 2, dans lequel les blocs dérivés du précurseur polymère ou du précurseur monomère sont rendus catianiques après greffage.

4. Copolymère greffé selon la revendication 2, dans lequel le mélange comprend un ou plusieurs monomères insaturés qui comprennent au moins un site d'insaturation éthylénique par molécule de monomère et au moins un groupe fonctionnel azoté par molécule de monomère.

5. Copolymère greffé selon la revendication 2, dans lequel le squelette du polymère de polysaccharide est dérivé d'un polymère choisi parmi les polygalactomannanes, les xanthanes, les polyfructoses, les amidons, les dérivés de l'amidon, la cellulose, les dérivés de la cellulose et des mélanges de ceux-ci.

6. Copolymère greffé selon la revendication 1, dans lequel le squelette du polymère de polysaccharide est dérivé d'une gomme de guar possédant un poids moléculaire moyen en poids avant le greffage d'environ 2 000 000 à environ 5 000 000 g/mole.

7. Copolymère greffé selon la revendication 1, dans lequel le polymère susceptible de se lier à des cheveux humains comprend un polymère cationique.

8. Copolymère greffé selon la revendication 1, dans lequel le polymère susceptible de se lier à des cheveux humains comprend un polymère essentiellement neutre choisi parmi les polymères de polyamide, les polymères de polyuréthane et les polymères de polyuréido.

9. Copolymère greffé selon la revendication 1, dans lequel le polymère susceptible de se lier à des cheveux humains comprend un polymère cationique choisi parmi les gommes de polysaccharide quaternisé.

10. Copolymère greffé selon la revendication 1, dans lequel le polymère susceptible de se lier à des cheveux humains comprend un polymère synthétique cationique choisi parmi les polymères d'addition cationique et les polymères de condensation cationique.

11. Copolymère greffé selon la revendication 1, dans lequel le polymère susceptible de se lier à des cheveux humains comprend un sel d'ammonium quaternaire polymère.

12. Procédé de préparation d'un copolymère greffé selon la revendication 1, comprenant :
le mélange du polymère de polysaccharide, du polymère susceptible de se lier à des cheveux humains et du polymère non susceptible de se lier à des cheveux humains ; et
l'irradiation du mélange de polymères avec une irradiation par faisceau d'électrons pour former le copolymère greffé.

13. Procédé selon la revendication 12, dans lequel le mélange de polymères est irradié avec une dose d'irradiation de 0,5 à 20 méga rads.

14. Composition capillaire, comprenant le copolymère greffé de la revendication 1 et un vecteur.

15. Composition capillaire selon la revendication 14, dans lequel le vecteur comprend l'eau.

16. Composition capillaire selon la revendication 14, dans laquelle la composition capillaire est choisie parmi les compositions de shampooing, les compositions d'après-shampooing et les compositions coiffantes.

17. Composition capillaire selon la revendication 14, dans laquelle la composition capillaire est un produit coiffant choisi parmi les gels coiffants, les mousses coiffantes et les sprays coiffants.

18. Composition, capillaire selon la revendication 14, dans laquelle la composition capillaire et une composition de shampooing ou une composition d'après-shampooing et comprend en outre un ou plusieurs tensioactifs.
